# EUROPEAN PATENT APPLICATION

(11) **EP 1 120 110 A1**
(43) Date of publication of application: **01.08.2001**
(21) Application number: 00830007.1
(22) Date of filing: 11.01.2000
(51) Int. Cl.: A61K 9/16, A61M 1/16

(54) **A composition for the preparation of dialytic solutions, a method for the production thereof, and a respective package**

(71) Applicant: Vasa di Sala Vittorio & C. S.n.c., 10015 Ivrea (Torino) (IT)
(72) Inventor: Sala, Vittorio, 10015 Ivrea (TO) (IT)
(74) Representative: Dragotti, Gianfranco

(57) **Abstract**

At least one granulate containing all of the ingredients or components of the final dialytic solution in predetermined and constant ratios is used for the instantaneous preparation of dialytic solutions, in line with the dialysis apparatus, so as to achieve uniform and controlled release of the individual ingredients into the final dialytic solution during the preparation thereof. Alternatively, two granulates are used to form, respectively, the acid solution and the basic solution which are to be mixed together, the granulates being prepared from mixtures of powders of suitable particle size, moistened with a concentrated aqueous humectant solution of one of the water-soluble salts constituting one of the two granulates.

As a further alternative, there are two or three granulates, one of which is constituted purely by the electrolytic salts constituting the final dialytic solution.

## Description

The invention relates to the preparation of dialytic solutions and, more specifically, to a composition for the preparation of these solutions, to a method for the production of the composition, as well as to types of packaging for these compositions.

It is well known that, during dialysis, a solution of known and controlled composition is passed in contact with a semi-permeable exchange membrane the other face of which is in contact with the bloodstream to be purified.

The dialytic solution must have a controlled composition (electrolytic salts, glucose, sodium bicarbonate) and is usually prepared by mixing two solutions, that is, an acid solution and a basic solution, respectively, with simultaneous dilution to bring each ingredient of the final dialytic solution to the desired concentration.

Normally, the starting solutions, in concentrated form, are held in containers from which they are withdrawn by means of metering pumps and, after the addition of diluting water, are admitted to a line for the preparation of the final solution, which line communicates with the supply of the dialysis apparatus.

So-called mixed systems which combine the use of a container holding a concentrated acid solution and a container which, on the other hand, contains bicarbonate powder, that is, bicarbonate in solid form, are also known and have found practical applications.

In this case, the physiological electrolytes, and possibly the desired quantity of glucose, are already included in the acid solution so that it suffices to pass a stream of water of suitable quality through the container holding the solid sodium bicarbonate, which is dissolved instantaneously, and to mix the resulting basic solution with the acid solution in the desired proportions to form the final dialytic solution.

The usual formulation of the two partial solutions (A and B) with which the dialytic solutions currently used are prepared are given below by way of illustration:

| Solution A | | | |
|---|---|---|---|
| | g | MW | nmoles/litre |
| NaCl | 172.20 | 58.44 | 2946.5 |
| KCl | 5.50 | 74.55 | 73.77 |
| CaCl₂.2H₂O | 9.48 | 146.49 | 64.49 |
| MgCl₂.6H₂O | 3.74 | 203.31 | 18.39 |
| CH₃COOH | 8.85 | 60.05 | 147.74 |

| Solution B | | | | |
|---|---|---|---|---|
| | g | MW | nmoles/1 | nmoles/1.83 1 |
| NaCl | 25.53 | 58.44 | 402.64 | 736.63 |
| NaHCO₃ | 65.93 | 84.00 | 785 | 1436 |

When the final dialytic solution is prepared, solutions A and B are mixed in the ratio of 1 litre of solution A to 1.83 litres of solution B and water is then added in sufficient quantity to give a final volume of 36.83 litres.

The composition of the final solution is thus as follows:

| | nmoles from A | nmoles from B | nmoles tot. | nmoles/l |
|---|---|---|---|---|
| Na⁺ | 2946 | 2172.83 | 5119.33 | 139 |
| K⁺ | 73.77 | | 73.77 | 2 |
| Ca⁺⁺ | 64.49 | | 64.49 | 1.75 |
| Mg⁺⁺ | 18.39 | | 18.39 | 0.5 |
| Cl⁻ | 3186.03 | 736.83 | 3922.86 | 106.5 |
| CH₃COO⁻ | 147.4 | | 147.4 | 4 |
| HCO₃⁻ | | 1436 | 1436 | 38.98 |

Many alternatives to these two main approaches to the technique for the preparation of dialytic solutions have been proposed without real success; for example, a technique consisting of the preparation of two cartridges containing, respectively, sodium bicarbonate in powder form and sodium chloride, also in powder form, as well as a bag containing a concentrated solution of the minor electrolytes has been proposed; the two solutions are prepared instantaneously by the supply of water to the two cartridges and are then mixed in the desired proportions with the simultaneous addition of a metered quantity of the solution of electrolytes taken from the bag.

As a further alternative, it has recently been proposed to use purely solid components, divided into at least two charges or bags; the respective aqueous solutions are prepared instantaneously and are then mixed in the desired proportions and the final solution is sent to the dialysis apparatus as usual.

In all of these cases, however, there remain problems which have not been solved satisfactorily and which can be summarized in the following points:
(1) with the conventional method, transportation of the containers holding the concentrated acid and basic solutions, respectively, involves the transportation of a considerable quantity of water which, of course, is expensive;
(2) containers, particularly in the form of bags, are often broken, naturally leading to wastage of materials and to the related costs;
(3) there is a problem with regard to the physical effort required by personnel employed in handling the bags or containers;
(4) there is the problem of the disposal of empty containers;
(5) finally, there is the problem of the timely preparation of the dialytic solutions, which cannot be kept for long periods.

Even in the above-mentioned alternatives, in which some of the above-mentioned disadvantages and problems are avoided, the problem of the preparation, upstream of the dialysis apparatus, of substantial volumes of solutions to be mixed in predetermined ratios still remains unsolved without considerable modification of the dialysis apparatus. Naturally, this latter possibility is not readily acceptable to users.

Moreover dialysis apparatus is arranged for the preparation of standardized dialytic solutions, whereas it would be optimal to prepare individualized dialytic solutions for each patient.

The main object of the present invention is substantially to solve the problems and disadvantages mentioned briefly above.

A more specific object of the present invention is to provide a composition in a suitable form for the instantaneous preparation of dialytic solutions, in line, ready for immediate use.

Another object of the present invention is to provide a method for the production of the compositions indicated above.

A further object of the invention is to provide specific types of packaging for the composition for the preparation of a dialytic solution, the packaging being compatible with currently existing dialysis apparatus without the need for substantial modification thereof.

Finally, yet another object of the invention is to provide a method for the preparation of the dialytic solution in line.

The main object of the invention is achieved by a composition for the preparation of dialytic solutions containing all of the components or ingredients known *per se* and used for the preparation of the acid solution and of the basic solution of the prior art, respectively, characterized in that the ingredients form part of at least one granulate in which the ingredients are present in predetermined and constant ratios.

In a first embodiment of the invention, all of the ingredients are included in a single granulate having a defined and constant composition so that the individual ingredients are released in a uniform and controlled manner, the granulate being usable directly for the preparation of the final dialytic solution, preferably in line.

In a second embodiment of the invention, the ingredients are divided into two granulates the composition of which preferably conforms to that of the two solutions of the prior art, that is, the acid solution and the basic solution.

Within the scope of this embodiment, the two granulates may be premixed with one another in predetermined ratios to form a single solid composition from which the final dialytic solution can be prepared directly, as in the previous embodiment or, alternatively, the two granulates are kept distinct and separate so that the two solutions, that is, the acid solution and the basic solution, respectively, are prepared from the two granulates and are then mixed to form the final dialytic solution.

According to a further embodiment of the invention, the ingredients are divided into two or three distinct granulates, of which one consists purely of the electrolytic salts and the other two are intended to form an acid solution and a basic solution.

In this case, during the preparation of the final dialytic solution, the corresponding solutions are prepared from the two or three granulates and are mixed to form the dialytic solution, or the acid solution and/or the basic solution serve to dissolve predetermined quantities of the third granulate.

It will readily be appreciated that this third embodiment may be very advantageous for the preparation of dialytic solutions which are individualized in accordance with the patient's therapeutic needs, since the electrolytic salts can be metered as well as selected both qualitatively and quantitatively.

During the preparation of the dialytic solution to be supplied to the dialysis machine, the quantity of granulate or granulates corresponding to the desired concentrations of ingredients are taken up, for example, by the sprinkling or passage of water, and the final dialytic solution is formed by the resulting solution or solutions, possibly further diluted.

Within the scope of the second embodiment of the invention, the two granulates may be kept separate so that the corresponding fraction of the final dialytic solution is prepared instantaneously from each of them or, even more preferably, the two granulates are mixed in predetermined ratios so that a single container is used upstream of the dialysis machine and contains the solid composition constituted by the two granulates, in contact with which the stream of water passes so that the dialytic solution is prepared continuously with constant monitoring of the concentrations of the individual ingredients.

It therefore becomes possible, with the present invention, to load a metered quantity of a single granulate or of granulates into a suitable section of the dialysis machine intended for the preparation of the dialytic solution and then to supply water to the respective container, instantaneously preparing the concentrated dialytic solution which is suitably diluted, in controlled manner, in the path along which it is supplied to the actual dialysis section.

The method for the production of the composition according to the invention in granulate form in turn provides for the steps of:
(a) grinding the required solid salts to a predetermined fineness;
(b) preparing a mixture containing predetermined quantities of the above-mentioned salts;
(c) preparing the final mixture from the premix and from remaining quantities of salts;
(d) moistening the final mixture of salts with an aqueous humectant solution to form a blend and granulating the blend;
(e) drying of the resulting granulate;
the method being characterized in that only a portion of the quantity of one of the salts, selected from those soluble in water, is used in the preparation of the final mixture and the remaining portion up to 100% of the quantity of the said salt is used for the preparation of the aqueous humectant solution for the step of blending and preparing the final granulate.

In the preferred embodiment of the invention, the salt used for the preparation of the humectant solution is selected from glucose, sodium acetate, sodium bicarbonate and sodium chloride.

The main advantage of the method defined above is that one of the ingredients of the final dialytic solution, provided it is soluble in water, is used for the preparation of the liquid humectant phase for moistening the mixture of salts in powder form in the conventional method of granulating powders.

In summary, a portion of the total glucose, sodium acetate or sodium chloride content of the dialytic solution is not used in the preparation of the starting mixture of powders but is used in the preparation of a concentrated aqueous solution with which the above-mentioned mixture of powders is wetted or moistened during the production of the corresponding granulate.

Bearing in mind that the composition of the dialytic solution is critical, it will readily be appreciated that a major variability factor of the above-mentioned composition is thus eliminated and a problem of primary importance is thus solved.

The method defined above is also applicable both to the preparation of a single granulate (that is according to the first embodiment of the invention) and to the preparation of two or more granulates.

The advantages which can be achieved by the present invention can readily be understood in terms both of uniformity of the characteristics of the dialytic solution instantaneously prepared and of the prevention of possible human errors in their preparation.

In addition, operations which are tiresome for personnel are eliminated and unnecessary costs for the transportation of water are avoided, at the same time also reducing storage costs.

The present invention also satisfactorily solves the problem of the stability of the dialytic solutions which, prior to the present invention, significantly affected the handling of the dialytic solutions; in fact the conventional solutions could not be kept for long periods, thus posing the problem of the re-supply of hospitals and other dialysis centres at short time intervals.

The present invention also relates to the packaging of the above-defined composition.

A first embodiment in fact consists of a single container holding predetermined quantities of both of the granulates constituting the composition, in predetermined ratios.

This container may be in the form of a rigid cartridge which is mounted directly on the dialysis apparatus in a position such as to be connected to a supply of water (or another suitable solvent) controlled, for example, by means of a metering pump; the connection may be performed in a manner such that the stream of water flows upwards from below or *vice versa.*

Alternatively, the container may be produced in the form of a bag of flexible material to be inserted as required in a rigid cylindrical casing mounted permanently on the dialysis apparatus and possibly having perforation means for perforating or opening the bag in some way, allowing the water to flow in contact with the granular material present therein. In this case also, the water may flow in one or other of the manners indicated above, according to choice.

In another embodiment, the two granulates are held separately in a single cartridge having a suitable partition so that water is supplied to both of the chambers defined by the partition in the cartridge and the two resulting solutions are combined downstream.

In all of the embodiments of the present invention, control members, for example, of the conductimetric type, are provided downstream of the stage in which the solution or the two partial solutions are prepared, for checking that the final dialytic solution conforms to the parameters required for the dialysis operation, these members in turn being connected to known means for modifying and/or supplementing one or more ingredients.

Finally, the present invention also enables compositions to be prepared according to the needs of individual patients, or groups of similar patients, since it suffices for the ingredients initially added to be modified appropriately, either qualitatively or quantitatively.

This advantage is very significant in the case of patients undergoing domiciliary dialysis since, in this case, it is precisely the accurate and controlled preparation of the dialytic solution which constitutes one of the major obstacles.

The present invention will now be described with reference to some examples of compositions with which experimental tests on the dissolution and preparation of dialytic solutions have also been performed.

With regard to the composition according to the present invention, with specific reference to the embodiment with two granulates, the formulations of the two solid portions of which the final composition was composed are given below by way of non-limiting example:

| Formulation I | | | |
|---|---|---|---|
| | g | MW | nmoles |
| NaCl | 172.20 | 58.44 | 2946.5 |
| KCl | 5.50 | 74.55 | 73.77 |
| CaCl₂.2H₂O | 9.48 | 146.99 | 64.49 |
| MgCl₂.6H₂O | 3.74 | 203.31 | 18.39 |
| CH₃COONa | 12.09 | 82.04 | 147.4 |

| Formulation II | | | |
|---|---|---|---|
| | g | MW | nmoles |
| NaCl | 43.06 | 58.44 | 736.83 |
| NaHCO₃ | 108.24 | 84.00 | 1288.6 |

These two formulations, in the dry or powdery state, were subjected to the granulation process defined above to produce granules each having a precise and controlled composition.

It is worth noting that formulation II was already calculated according to the quantity of ingredients which should be mixed with the unitary quantity of formulation I in the final dialytic solution. In other words, in the case of solutions A and B of the prior art, the two solutions had to be mixed in the ratio of 1 litre of solution A to 1.83 litres of solution B, with further provision for the addition of diluting water to give the desired final volume.

In the case of the present invention, however, equal quantities of the two formulations I and II are taken and mixed, with the addition of water in sufficient quantity to give a final solution with a volume of 36.96 litres.

The final dialytic solution thus had the following composition:

| | nmoles from I | nmoles from II | nmoles tot. | nmoles/l |
|---|---|---|---|---|
| Na⁺ | 3093.9 | 2025.43 | 5119.33 | 139 |
| K⁺ | 73.77 | | 73.77 | 2 |
| Ca⁺⁺ | 64.49 | | 64.49 | 1.75 |
| Mg⁺⁺ | 18.39 | | 18.39 | 0.5 |
| Cl⁻ | 3186.03 | 736.83 | 3922.86 | 106.5 |
| CH₃COO⁻ | 147.4 | | 147.4 | 4 |
| HCO₃⁻ | | 1288.6 | 1288.6 | 34.99 |

As indicated above, the present invention also permits and provides for the two formulations I and II, already mixed in the solid state, to be packaged in a single container to be loaded into the dialysis apparatus at the time required, in the section dedicated to the instantaneous preparation of the dialytic solution as required.

Moreover, no glucose content has been indicated in the formulations given above, but glucose may be added to one or other formulation since this presents no problems from the dissolution point of view.

In order to check the behaviour of the solid formulations according to the present invention, dissolution tests were performed, in the course of which the behaviour of the formulations was checked according to whether the flow of water was directed upwards from below or *vice versa.*

For this specific test, a single formulation having the composition given below and substantially corresponding to that of formulation I was used, except that glucose was added and the sodium acetate was omitted:

| Formulation III | | |
|---|---|---|
| | g | nmoles/1 (as ions) |
| NaCI | 284.33 | 139 (Na⁺) |
| KCl | 5.22 | 2 (K⁺) |
| CaCl₂.2H₂O | 7.72 | 1.5 (Ca⁺⁺) |
| MgCl₂.6H₂O | 2.63 | 0.37 (Mg⁺⁺) |
| | | 144.74 (Cl⁻) |
| Glucose monohydrate 35 | | 1 g/l |

In the test, 340 grams of the granulate of formulation III, corresponding to 296.71 g of anhydrous salts, were used and were loaded into a glass column having the following dimensional characteristics:

| | |
|---|---|
| diameter | 5 cm |
| cross-section | 20 cm² |
| height | 33 cm |
| column volume | 660 cm³ |
| height of granular product | 17 cm |
| volume of granular product | 340 cm³ |

For the dissolution test, the water was supplied at a flow-rate of 13.4 ml/min. In the cycle with water supplied from the bottom of the column, the cycle time was 88 minutes so that the volume of water supplied was 1179 ml.

It was found that, 15 minutes after the start of the supply of water, the water had reached the top of the layer of granular product (corresponding to 210 ml of water).

After 40 minutes, the column of granular product was reduced to a volume of about 50% of the initial volume, corresponding to 560 ml of water supplied.

When 48 minutes had elapsed, the water had reached the top of the column and its quantity was 643 ml.

After 65 minutes, the granular product was completely dissolved and the quantity of water supplied was 871 ml (some adhered to the walls of the column for a height of about 3 cm.).

The initial time (T=0) was set at the moment at which the water started to emerge from the column and the test was continued for 70 minutes with samples taken every 10 minutes.

Table 1 below gives the saline composition detected in the course of the dissolution at some of the sampling times and Table 2 gives the quantities of salts recovered at the various times, compared with standard values.

**Table 1**

| t (min) | Na [g/l] | K [g/l] | Mg [g/l] | Ca [g/l] | NaCl [g/l] | KCl g/l] | MgCl₂ [g/l] | CaCl₂ [g/l] |
|---|---|---|---|---|---|---|---|---|
| 0 | | | | | | | | |
| 10 | 97.6 | 2.655 | 0.24 | 1.795 | 247.99 | 5.08 | 2.03 | 6.60 |
| 20 | 97.6 | 2.75 | 0.24 | 1.927 | 246.46 | 5.25 | 2.05 | 7.08 |
| 40 | 93.8 | 2.79 | 0.25 | 1.935 | 238.33 | 5.34 | 2.11 | 7.11 |
| 70 | 69 | 1.1135 | 0.021 | 0.835 | 175.32 | 2.13 | 0.18 | 3.07 |
| | 2.5408696 | 1.9125641 | 8.45833 | 3.675 | | | | |

**Table 2**

| t [min] | NaCl [g] | KCl [g] | MgCl₂ [g] | CaCl₂ [g] | Tot. [g] |
|---|---|---|---|---|---|
| 0 | | | | | |
| 10 | 33.23 | 0.68 | 0.27 | 0.88 | 35.07 |
| 20 | 33.02 | 0.70 | 0.28 | 0.95 | 34.35 |
| 40 | 63.81 | 1.43 | 0.56 | 1.91 | 67.71 |
| 70 | 70.48 | 0.86 | 0.07 | 1.23 | 72.64 |
| Tot. [g] | 200.54 | 3.67 | 1.18 | 4.98 | 210.37 |
| Start. [g] | 284.33 | 5.22 | 1.23 | 5.83 | 296.61 |
| Tot. [%] | 95.33% | 1.74% | 0.56% | 2.37% | |
| Start. [%] | 95.86% | 1.76% | 0.41% | 1.97% | |

The test was repeated with the same composition except that the water was supplied from the top of the glass column instead of from the bottom. In this case also, samples were taken every 10 minutes for 70 minutes starting from time T=0.

Tables 3 and 4 below, which correspond to Tables 1 and 2, provide the data measured.

**Table 3**

| t (min) | Na [g/l] | K [g/l] | Mg [g/l] | Ca [g/l] | NaCl [g/l] | KCl [g/l] | MgCl₂ [g/l] | CaCl₂ |
|---|---|---|---|---|---|---|---|---|
| 0 | | | | | | | | |
| 10 | 91.2 | 4.725 | 0.46 | 2.975 | 231.73 | 9.04 | 3.89 | 10.93 |
| 20 | 94.5 | 3.08 | 0.32 | 2.78 | 240.11 | 5.89 | 2.71 | 10.22 |
| 40 | 78.8 | 2.79 | 0.025 | 0.17 | 200.22 | 5.34 | 0.21 | 0.62 |
| 70 | 73.6 | 0.02 | 0.015 | 0.014 | 187.01 | 0.04 | 0.13 | 0.05 |
| | 2.5408696 | 1.9125641 | 8.45833 | 3.675 | | | | |

**Table 4**

| t [min] | NaCl [g] | KCl [g] | MgCl₂ [g] | CaCl₂ [g] | Tot. [g] |
|---|---|---|---|---|---|
| 0 | | | | | |
| 10 | 31.05 | 1.21 | 0.52 | 1.47 | 34.25 |
| 20 | 32.18 | 0.79 | 0.36 | 1.37 | 34.70 |
| 40 | 53.66 | 1.43 | 0.06 | 0.17 | 55.31 |
| 70 | 75.18 | 0.02 | 0.05 | 0.02 | 75.26 |
| Tot. [g] | 192.06 | 3.45 | 0.99 | 3.02 | 199.52 |
| AFB [g] | 284.33 | 5.22 | 1.23 | 5.83 | 296.61 |
| Tot. [%] | 96.26% | 1.73% | 0.50% | 1.51% | |
| AFB [%] | 95.86% | 1.76% | 0.41% | 1.97% | |

It can be seen from the results given above that the resulting solution had a percentage concentration of the various ingredients which, for a large part of the dissolution period, and hence of the preparation of the dialytic solution, conformed to the percentage ratios of the same ingredients in the starting solid. Only towards the conclusion of the dissolution of the salts were there significant variations in the concentrations of the ingredients, which can easily be compensated by modification of the additions of diluting water.

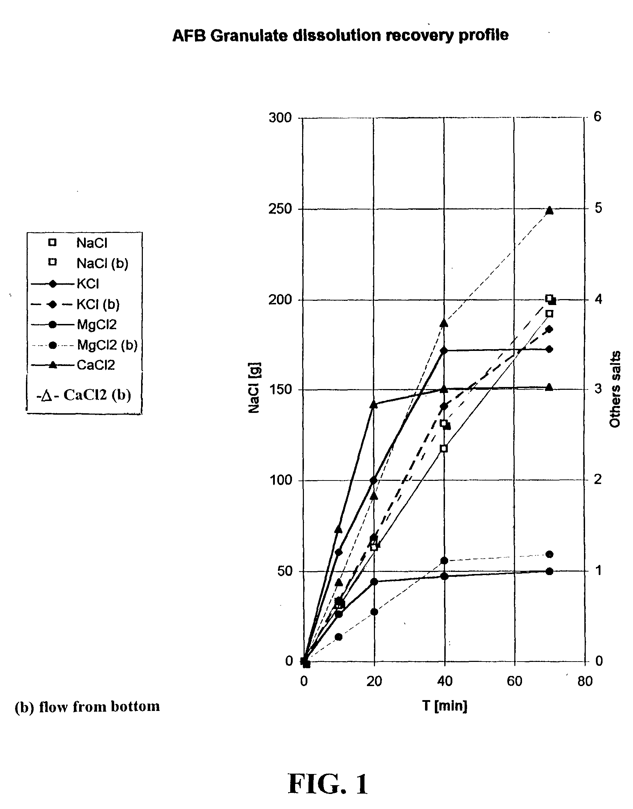

The values given in the tables are shown in graphical form in appended Figure 1 (in which the letter (b) accompanying the formula of the salt indicates supply from the bottom) and it can readily be seen that, in both cases, the saline composition of the resulting solution was satisfactory. However, supply from the bottom seems preferable. It is worth noting that the conditions of the dissolution test described above were actually more critical and severe than the normal conditions for the preparation of the dialytic solution. In fact, since the glass column was filled with granulate for approximately half of its height, rather than completely, twice the volume of water was required to move the front of the solute towards the upper end of the column and this could lead to variations in composition due to dilution of the solution.

When the glass column is completely filled with granulate, however, a saturated solution is formed and continues to flow from the column until the whole of the volume of granulate present in the column has been displaced, so that the effect of the dilution is very small.

With regard to the production of the solid compositions of the present invention, some examples are now given by way of non-limiting illustration.

### Example 1

In order to produce 200 kg of granulate having the composition of formulation III indicated above, the following solid ingredients were provided:

| | |
|---|---|
| NaCl | 168.79 kg |
| KCl | 3.11 kg |
| CaCl₂.2H₂O | 4.61 kg (equal to 3.52 kg of anhydrous salt) |
| MgCl₂.6H₂O | 1.57 kg (equal to 0.74 kg of anhydrous salt) |
| glucose monohydrate | 21.90 kg |

These ingredients were ground finely in a mill having a screen with 0.5 mm holes and then sieved on a screen with 520 meshes/cm².

A premix comprising KCl, CaCl₂ and MgCl₂ and 11.90 kg of glucose monohydrate was prepared in a mixer with blades.

After mixing for 5 minutes, this premix was loaded into a mixer of suitable capacity together with one half of the sodium chloride and mixing was continued for 5 minutes. The other half of the NaCl was then added and mixing was continued for 10 minutes.

The remaining 10 kg of glucose monohydrate was dissolved in 7.00 litres of distilled water in a separate container.

The resulting solution was added slowly and in portions to the dry mixture previously produced and the resulting mixture was subjected to the mixing action for approximately 20 minutes.

The final blend was passed through a screen with 16 meshes/cm² and the resulting granules were dried in an oven at 50°C for 10 hours.

Finally, the final granulate was standardized on a screen with 36 meshes/cm².

Examples 2 and 3 below, on the other hand, relate to the preparation of a single granulate in accordance with the first embodiment of the invention defined above.

### Example 2

Example 1 was repeated, again with reference to the preparation of a 200 kg batch, with the following ingredients:

| | |
|---|---|
| NaCl | 121.5 kg |
| KCl | 3.10 kg |
| CaCl₂.2H₂O | 5.35 kg (equal to 4.09 kg of anhydrous salt) |
| MgCl₂.6H₂O | 2.11 kg (equal to 1.00 kg of anhydrous salt) |
| CH₃COONa.3H₂O | 112.76 kg (equal to 67.94 kg of anhydrous salt) |

The preparation was performed as in Example 1 except that, instead of the glucose monohydrate, 6.50 kg of sodium acetate and 5.50 litres of distilled water were used for the preparation of the humectant solution for the production of the granulate.

### Example 3

For the preparation of a 200 kg batch of granulate, the solid starting ingredients were as follows:

| | |
|---|---|
| NaCl | 128.80 kg |
| KCl | 3.21 kg |
| CaCl₂.2H₂O | 5.54 kg (equal to 4.09 kg of anhydrous salt) |
| MgCl₂.6H₂O | 2.19 kg (equal to 1.00 kg of anhydrous salt) |
| NaHCO₃ | 63.26 kg |

The method of Example 1 was repeated, except that, instead of the glucose monohydrate, 3.00 kg of sodium chloride and 6.00 litres of distilled water were used to prepare the humectant solution for the granulation stage.

The same method was also followed for the preparation of two or three separate granulates and, in this case, the granulate constituted exclusively by electrolytic salts was prepared with the use, as the humectant or blending agent, of a portion of the total quantity of a water-soluble salt selected from the electrolytes or from the other components of the composition, the quantity of this same salt in one or both of the other two granulates being correspondingly reduced.

The present invention has characteristics of particular versatility and adaptability to many different situations.

In fact, by using entirely solid ingredients and particularly granulates (so as to have constant ratios of the individual components in each granule), it becomes possible to select different methods for the preparation of the dialytic solutions. In particular:
(1) in the case of a single granulate, the quantities of the individual components released into the final dialytic solution are in predetermined and constant ratios;
(2) in the case of two granulates, by keeping them apart, it is possible, to prepare the two solutions in order then to mix them as is done at the moment, but with the two solutions being prepared at the time required and being metered in a manner such that the mixing of equal volumes produces the desired final composition;
(3) the solution generated by the granulate which is to form the acid solution is used as a solvent phase for the granulate which is to form the basic solution;
(4) the two granulates are packaged in a single container which in turn may have an internal partition for keeping them apart, or the two solid phases are mixed homogeneously so that the final dialytic solution is obtained from the container;
(5) as well as being packaged in granulate form, the solid powders may naturally also be packaged in the form of pellets or micro-tablets, as well as in the well-known delayed-release formulations;
(6) it is not necessary to modify existing dialysis apparatus since the preparation of the final dialytic solution with the granulate or granulates of the present invention takes place upstream of and in line with the dialysis apparatus;
(7) all of these alternatives are clearly also possible with two or three granulates, as defined above.

With regard to the latter aspect, in-line preparation with the compositions and packages according to the present invention provides for the following possibilities:
(1) if the two granulates which are to form the two solutions, that is, the acid solution and the basic solution, respectively, to be mixed in the final dialysis solution, are kept separate and hence in respective chambers (irrespective of whether these two chambers are parts of a single container or cartridge or consist of two physically separate containers), each chamber is supplied with water of suitable quality and with the desired flow-rate by means of a dedicated metering pump, with a flow control and a composition control (for example, in the form of conductimetric cells) connected downstream of the chamber (and hence at the outputs of the acid solution and of the basic solution thus prepared, respectively); the two solutions are then combined in equal volumes and supplied to the dialysis apparatus;
(2) if a single initial mixture is formed with two or three granulates or if a single granulate is used, the chamber or cartridge which contains it is supplied with water by means of a metering pump and the resulting solution supplies the dialysis apparatus, with the flow control and the composition control kept downstream of the cartridge.

The invention has been described with reference to preferred embodiments thereof, upon the understanding that conceptually equivalent modifications and variations are possible and are to be expected, without departing from its scope.

## Claims

1. A composition for the preparation of dialytic solutions which contains all of the components or ingredients known *per se* and used for the preparation of the acid solution and the basic solution of the prior art, respectively, characterized in that the ingredients form part of at least one granulate in which the ingredients are present in predetermined and constant ratios.

2. A composition for the preparation of dialytic solutions according to Claim 1, characterized in that all of the ingredients of the final dialytic solution form a single granulate in which each granule has a well-defined and constant composition, in which the ratios between the individual ingredients are predetermined and constant and their release takes place in a uniform and controlled manner.

3. A composition for the preparation of dialytic solutions according to Claim 1, characterized in that it consists of two granulates, of which a first, corresponding to the basic solution of the prior art, is preferably constituted by sodium chloride and bicarbonate, and the second granulate, corresponding to the acid solution of the prior art, contains the other required ingredients of the dialytic solution.

4. A composition for the preparation of dialytic solutions according to Claim 1, characterized in that it consists of two granulates of which a first is constituted by all of the electrolytic salts required in the final dialytic solution and the other granulate is constituted by the other components of the final dialytic solution.

5. A composition for the preparation of dialytic solutions according to Claim 3, characterized in that, amongst the ingredients of the dialytic solution, the second granulate contains, in particular, sodium acetate.

6. A composition for the preparation of dialytic solutions according to Claim 3, characterized in that the two granulates are kept separate until the respective acid and basic solutions are prepared in line, the acid and basic solutions then being mixed in the desired ratios, forming the final dialytic solution to be supplied to the dialysis apparatus.

7. A composition for the preparation of dialytic solutions according to Claim 3, characterized in that the two granulates are mixed in predetermined ratios and the single resulting granulate is used directly for the preparation of the dialytic solution to be supplied to the dialysis apparatus.

8. A composition for the preparation of dialytic solutions according to Claim 7, characterized in that the single granulate is used for the preparation of concentrated dialytic solution which is diluted, again in line, with water of suitable quality to produce the desired concentrations.

9. A composition for the preparation of dialytic solutions according to Claim 1, characterized in that it consists of three granulates, of which a first is constituted by all of the electrolytic salts required in the final dialytic solution and the other two granulates are constituted by components which are intended to form an acid solution and a basic solution, respectively.

10. A method for the production of the composition according to Claim 1 in granulate form, which provides for the steps of:
(a) grinding the required solid components to a predetermined fineness;
(b) preparing a premix containing predetermined quantities of the above-mentioned components;
(c) preparing the final mixture from the premix and from remaining quantities of the components;
(d) moistening the final mixture of components with an aqueous humectant solution to form a blend and granulating the blend;
(e) drying the resulting granulate;
the method being characterized in that only a portion of the quantity of one of the components, selected from those soluble in water, is used in the preparation of the final mixture and the remaining portion up to 100% of the quantity of the said component is used for the preparation of the aqueous humectant solution in the step of blending and preparing the final granulate.

11. A method of producing granulate according to Claim 10, characterized in that the component used for preparing the aqueous humectant solution is selected from glucose, sodium acetate and sodium chloride.

12. A package for the composition in granulate form of any of Claims 1 to 9, characterized in that it consists of a container holding predetermined quantities of the at least one granulate.

13. A package according to Claim 12, characterized in that the container is divided into two or into three separate chambers, each of which contains one of the two or of the three granulates, respectively.

14. A package according to Claim 12, characterized in that the container is constituted by a single chamber containing the granulates, mixed in predetermined ratios.

15. A package according to Claim 13 or Claim 14, characterized in that the granulates are contained in non-rigid bags to be inserted in the chamber or chambers, releasing their contents therein.
